# EUROPEAN PATENT SPECIFICATION

(11) **EP 1 214 129 B1**
(45) Date of publication and mention of the grant of the patent: **19.07.2006**
(21) Application number: 00951767.3
(22) Date of filing: 18.08.2000
(51) Int. Cl.: B01D 9/00, B01F 5/02, A61K 9/00

(54) **PROCESS FOR PRODUCING FINE MEDICINAL SUBSTANCES**
VERFAHREN ZUR HERSTELLUNG VON FEINKÖRNIGEN MEDIZINISCHEN SUBSTANZEN
PROCEDE DE PRODUCTION DE SUBSTANCES MEDICINALES FINES

(30) Priority: 19.08.1999 GB 9919693
(43) Date of publication of application: 19.06.2002
(73) Proprietor: Aventis Pharma Limited, West Malling, Kent ME19 4AH (GB)
(72) Inventor: BEGON, Dominique, 69153 Decines Charpieu Cedex (FR); GUILLAUME, Pfefer, 69153 Decines Charpieu Cedex (FR); KOHL, Michael, 69153 Decines Charpieu Cedex (FR)
(74) Representative: Jones, Stephen Anthony
(86) International application number: PCT/GB2000/003178
(87) International publication number: WO 2001/014036

(56) References cited:
- WO-A-00/53282
- WO-A-94/07582
- WO-A-95/05805
- WO-A-96/32095
- GB-A- 741 756
- US-A- 3 897 779
- US-A- 5 314 506

## Description

Medicaments designed for. administration in solid form must meet a number of physical criteria. Important criteria which can control how a drug is absorbed into the body after administration are particle size and shape and crystalline form. For inhalation use, the primary requirement is that medicament be delivered to the lung with the correct range of particle size. Typically this lies within 1 and 10 micrometer. Within this size range particles can penetrate deep within the lung and be effectively absorbed. Finer particles are normally exhalable and coarser particles are caught in the nose, mouth and throat during inhalation.

Typically, the final stage of production of medicament compounds involves a crystallisation from solution. The conditions used are chosen to give good chemical purity with minimum solvent containment and this usually produces relatively large crystals which are outside the optimum size range mentioned above. In order to reduce particle size to the correct range the product is usually milled. A number of standard milling techniques are available, the most common in the pharmaceutical industry being fluid energy milling or micronisation. Dry milling is commonly used because it can be difficult to dry a slurry of fine powder without producing hard agglomerates.

When a powder is dry milled to below 10 micrometer a considerable amount of specific energy is required, which produces a powder with a significant proportion of particles below 1 micrometer and also tends to produce a significant proportion of amorphous material on the particle surfaces. This can have the effect of rendering the powder susceptible to agglomeration, often due to moisture pick up, or to dissolution in aerosol propellant used in a metered dose inhaler.

The problems associated with dry milling have been recognised in the prior art. WO-A-92/18110 and WO-A-95/05805 describe methods of treating milled powders to reduce these problems. WO-A-92/18110 describes drying the milled powder to remove residual water, treating with an organic solvent, and a further treatment to remove the solvent. WO-A-95/05805 describes treating the powder with water vapour under controlled conditions to reduce the amorphous content.

An additional problem with pharmaceutical powders is that many medicaments are soft waxy solids and such materials are difficult to mill and difficult to prevent from re-agglomerating. This is a particular problem with steroid based drugs, many of which are used in inhalation therapy.

An alternative to milling is to form particles of the correct particle size by direct precipitation from solution. However, this process also has problems. Thus, for example, WO-A-95/05805 describes treating precipitated fine medicament powders for problems similar to those associated with milled powders. In general in order to form a fine precipitate by crystallisation the crystallisation process must be rapid. Unfortunately, rapid crystallisation is often accompanied by entrainment of impurities in the crystals formed. Also, the process can be difficult to control to give a uniform and reproducible particle size distribution. This applies to processes where precipitate is formed by rapid cooling of a saturated solution and to processes where a solution is mixed with anti-solvent.

US-A-5 314 506 discloses a method of producing relatively fine crystalline organic medicament particles which is claimed to overcome some of the problems identified above. The process comprises forming a solution of the medicament in a suitable solvent and passing the solution through a jet nozzle or nozzles at a controlled rate of flow. An anti-solvent is passed through an opposing nozzle or nozzles, thereby forming a region of intense mixing. The slurry of fine medicament is passed into a holding tank and the fine powder recovered by conventional filtration and/or drying techniques. Crystalline powders with a particle size substantially all less than 25 micrometer are claimed to be produced, and the incorporation of solvent inclusions as an impurity is said to be less than for conventional precipitation techniques.

However, this patent is primarily concerned with producing crystalline drug substances suitable for oral administration rather than inhalation medicaments.

The present invention relates to microprecipitation techniques to produce fine medicament powders, particularly fine medicament powders suitable for administration by inhalation. Any medicament suitable for administration via the lung can be treated by this process, but the process is especially applicable to steroid based medicaments which can be difficult to mill by conventional processes, and in particular to triamcinolone acetonide.

The compound to be precipitated is first dissolved in a suitable solvent to give a 'medicament solution'. A suitable solvent is one which is capable of producing a reasonably concentrated solution of compound and which is miscible with another liquid in which the compound is insoluble or only sparingly soluble, referred to herein as the 'anti-solvent'. Suitable solvents are water miscible alcohols, esters, ethers or amides. Dimethylformamide is a particularly suitable solvent. The anti-solvent is most commonly water.

The microprecipitation step is preferably carried out in a continuous fashion. A flow of medicament solution and a flow of anti-solvent are generated through two or more nozzles; the streams impinging upon each other in a small chamber to generate a region of extreme turbulence and intense mixing. The combined solutions, referred hereinafter as the 'mixed suspension', exits the mixing chamber into a holding chamber. The relative flow rates of medicament solution and anti-solvent are chosen to produce a supersaturated condition in the mixed suspension which causes the compound to precipitate.

If desired the medicament solution, or more preferably the anti-solvent, may contain a small proportion of previously crystallised compound to act as seed. If the medicament has any solubility in the anti-solvent it may be advantageous to saturate the anti-solvent with medicament before use. Alternatively, or in addition, one or other or both of the medicament solution and anti-solvent may contain additives to aid crystallisation or modify crystal habit or improve handlability of the slurry formed. Suitable additives include surfactants which will help stabilise the mixed suspension. Since all such additives are likely to become incorporated into the medicament powder they should be used at the minimum levels possible and should be acceptable for administration to the human or animal body.

The streams may impinge on each other at any angle provided sufficient mixing is generated. However, it is preferred that the angle between the streams is between 0°, when they are directly opposed, and 90°. Configurations where the angle between the streams is less than 20° are preferred, particularly configurations where the streams are substantially directly opposed.

Thus far the process has similarities to that described in US-A-5 314 506. However, we have found that this process does not always produce powders with the uniform properties that may be required, especially for inhalation use. The precipitate produced may have a broad particle size distribution, often appearing to be a mixture of coarse and fine fractions. Also we have found that medicament particles may have relatively high contents of solvent and that such particles perform less well in the final application. For example, the respirable fraction produced by a drug powder with a higher content of absorbed solvent is generally significantly lower than that from a similar powder with low levels of absorbed solvent.

There are a number of parameters which control the quality of the product produced. We have found that control of particle size distribution (psd) of the precipitated product is critically dependant on close control of the velocities of the opposed streams of medicament solution and anti-solvent. We have found it necessary to use somewhat higher velocities than those exemplified in US-A-5 314 506. The exact velocities to be used will depend on the nature of each of the medicament, solvent and anti-solvent. A balance must be struck between higher relative velocities which tend to give finer psd but higher residual solvent levels and lower relative velocities which tend to give the opposite effects.

Any variation in velocity of either or both streams causes a variation in psd. The cyclic variation which can be caused if pumps are used to generate the streams may be enough to produce the broad/double size distribution referred to above. In order to reduce this effect to acceptable levels anti pulsation devices are required on the pumps. It has also been observed that cyclic variations in the velocity of either or both streams leads to higher levels of residual solvent becoming entrapped in the crystal structure of the precipitated product.

Another.important parameter is the relative volume of medicament solution and anti-solvent used. We have found that best results are obtained when a large excess of anti-solvent is used. We have also found that the concentration of the medicament solution used is an important parameter. A low concentration tends to give a lower concentration of residual solvent in the product but also tends to give a larger psd. Increasing the concentration tends to give a lower psd but higher residual solvent levels.

Thus, according to the present invention, there is provided a method of producing medicament particles comprising dissolving the medicament in a solvent, producing one or more streams of medicament solution and contacting these streams with one or more streams of anti-solvent in order to produce a region of turbulent mixing in which rapid precipitation of medicament crystals takes place wherein the ratio of the volume flow of anti-solvent to volume flow of medicament solution exceeds 2:1 and the velocity of each stream is controlled to substantially remove cyclic variations.

The suspension leaves the mixing chamber and is conveyed to a holding tank for further processing. Whilst being conveyed the crystals are forming and ripening. It can be advantageous if the suspension is conveyed in such a manner that turbulence in the conveying line is reduced as much as possible.

Once the suspension has been produced it can be treated by conventional techniques to produce a fully crystalline dry powder. It may be subjected to ultrasonic agitation either immediately after precipitation or during further processing. Because the product has no amorphous material on the surface of the particles the problems of solvent or anti-solvent entrapment and agglomeration on drying are much reduced. Intensive milling of the dried powder is not required although a gentle milling process may be used to break down any agglomerated particles.

According to the method of claim 1 a substantially fully crystalline medicament suitable for inhalation is obtained. Preferably the medicament has a particle size of between 1 and 10 micrometer, more preferably 1-7 micrometer and most preferably between 2 and 5 micrometer. Such a medicament is characterised by comprising substantially uniform, smooth crystalline particles. Medicament produced by this precipitation process has a high respirable fraction when measured by conventional techniques and delivers an improved respirable dose when used in a multidose dry powder inhaler in conjunction with conventional carrier powders. What constitutes an acceptable respirable fraction in this context depends on the drug used and, to some extent, on the method of delivery. However, in general, fine powders produced by the above process give higher respirable fractions than fine powders of the same medicament produced by conventional techniques.

A further aspect of this invention provides an apparatus for carrying out the precipitation process described above comprising a cylinder with two or more orifices set into the cylinder walls in opposition to each other through which streams of medicament solution and anti-solvent are produced which impinge on each other and pumps which produce the streams of medicament solution and anti-solvent and wherein the apparatus comprises means for reducing cyclic variations in stream velocities.

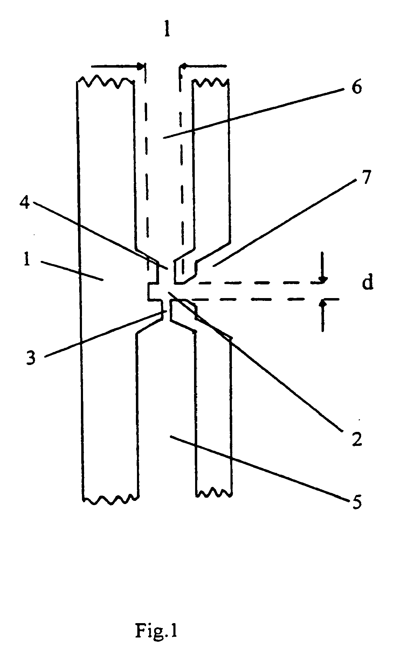

A preferred form of apparatus for carrying out the method of the invention is shown in Figure 1. It comprises a metal block (1) with a cylindrical bore (2) with an internal diameter d and a length 1. Two orifices, (3) and (4) are set into the cylinder wall directly opposing each other and arranged to provide streams of liquid which contact each other. The liquid is fed to the orifices under pressure via feed pipes (5) and (6). Preferably, the streams of liquid are at right angles to the axis of the cylinder. The orifices may be proud of the cylinder wall or set into it provided only that the streams of liquid emanating from them are uninterrupted until they hit each other. Orifice (3) dispenses medicament solution and has a diameter Dₛ. Orifice (4) dispenses anti-solvent and has a diameter Dₐ. The two diameters may be the same or different. After mixing the output from the streams passes through the cylinder (2) via outlet (7) into a holding tank from which the precipitated powder is washed and separated from the mixed solvent and anti-solvent by conventional means.

The streams of liquid are generated by hydrostatic pressure using any conventional means. A separate generator is conveniently used for each feedstock. It is essential that the pressure applied be controlled throughout the process. A convenient method of generating pressure is a pump.

The velocity of each stream is controlled to remove any cyclic variation. This most commonly arises if a pump is used to generate the pressure needed to produce one or more of the streams of liquid. Many pumps generate pressure by a rotary or reciprocal motion and it is common for the pressure generated to vary in a cyclic fashion due to this action. Such cyclic variations in pressure may lead to cyclic variations in the velocities of the streams. This in turn may lead to cyclic variation in the particle size of the crystallites produced by the process. Due to mixing in the holding tank this will manifest itself in a broadening of the particle size distribution of the product.

Therefore, if a pump or pumps are used to generate the streams they should be fitted with anti-pulsation equipment.

Normally only one stream each of medicament solution and anti-solvent will be used. However, the process may be operated using two or more streams of each provided that the orifices are symmetrically arranged around the cylinder circumference, that each set of orifices has the same diameter, and that the flow through each orifice is the same.

In principle the dimensions d, l, Dₛ and Dₐ may have any convenient value consistent with achieving the required flow rates and micromixing turbulence. We have found that relatively small dimensions give the best results and that it is better to scale up production by increasing the number of microprecipitation units rather than their size. Diameter d is preferably between 0.1 and 2mm, more preferably 0.2 to 1mm and most preferably around 0.5mm. Length 1 is less critical and may typically be between 0.5 and 10 mm, preferably about 0.7mm. Dₛ is preferably between 50 and 200 micrometer, more preferably between 80 and 150 micrometer and most preferably around 100 micrometer. Dₐ is preferably between 100 and 500 micrometer, more preferably between 200 and 400 micrometer and most preferably around 300 micrometer.

The range of flow rates of medicament solution and anti-solvent depend on the dimensions of apparatus used. For the dimensions given above the flow rate of medicament solution is preferably between 2 and 40ml/min, more preferably between 5. and 30ml/min and most preferably 10 to 20ml/min. The flow rate of anti-solvent is preferably between 4 and 1000ml/min, more preferably between 10 and 600 ml/min and most preferably between 100 and 400 ml/min. As described above an excess of anti-solvent is needed and the ratio of flow rate of anti-solvent to medicament solution must be greater than 2:1, preferably greater than 10:1 and more preferably between 15:1 and 30:1.

The stream velocities produced depend on the combination of flow rate and orifice diameter used. The minimum relative stream velocity (i.e.. the sum of the vectors of the velocity of solution and anti-solvent which oppose each other) required to be effective is 50m/s. Preferably, the relative stream velocity should be between 70 and 200m/s.

The method of this invention may be applied to any medicament for which suitable solvents and anti-solvents are available. It is particularly applicable to steroids such as triamcinolone acetonide (TAA).

### Example 1

A microprecipitation cell with a diameter of 0.5mm was used. TAA was dissolved in dimethylformamide (DMF) at a concentration of 250g/l. The anti-solvent was water. The process was carried out at room temperature. TAA solution was forced through a 100 micrometer orifice by a pump with an anti-pulsing device at a rate of 14ml/min, giving a stream velocity of 30m/s. Water was forced through a 300 micrometer orifice by a similar pump to give a flow rate of 333ml/min and a stream velocity of 79m/s. The process was continued for approximately one hour to provide 200g dry weight of product.

The resulting slurry was agitated for 4 hours in a holding vessel before being vacuum filtered, washed and freeze dried. The median particle size of the powder was 1.9 micrometer as measured by Malvem particle sizer. 80% of the particles were within the size range of 0.8 to 4.4 micrometer. The powder produced was mixed with lactose carrier, compressed into a medicament compact, and filled into an Ultrahaler^{R} multidose dry powder inhaler. The respirable fraction produced from the Ultrahaler^{R} was 44%

### Comparative example

The experiment described above was repeated using the same apparatus and ingredients but with a flow rate of 7.5m/s for TAA solution and 20m/s for water. Approximately 50g dry weight of product was produced. The pumps were not fitted with anti-pulsing devices.. The median particle size of the powder was 3.3 micrometer with 80% of the particles within the size range of 0.8 to 7.2 micrometer. The respirable fraction provided by the Ultrahaler^{R} was 27%.

### Example 2

A microprecipitation cell with a diameter of 0.4mm was used. The solution orifice was 100 micrometer in diameter and the anti-solvent orifice was 300 micrometer in diameter. TAA was dissolved in dimethylformamide (DMF) at a concentration of 250g/l. The anti-solvent was water. The process was carried out at room temperature. Four runs were carried out at high and low flow rates and with and without anti-pulsating devices fitted. The process was continued for approximately one hour to provide 200g dry weight of product.

The resulting slurry was agitated for 4 hours in a holding vessel before being vacuum filtered, washed and freeze dried. The median particle size of the powder was measured by Malvern particle sizer. The powder produced was mixed with lactose carrier, compressed into a medicament compact, and filled into an Ultrahaler^{R} multidose dry powder inhaler. The Ultrahaler® is a dry powder inhaler whose basic operation is described in EP 407 028. The respirable fraction produced from the Ultrahaler^{R} was measured.

Results are tabulated below: -

| Relative Stream Velocity ms⁻¹ | Anti-pulsing | Median Particle Size Micrometer | Respirable Fraction % | Residual Solvent % |
|---|---|---|---|---|
| 30 | Yes | 2.2 | 44.5 | 680 |
| 30 | No | 3.9 | 35.6 | 1220 |
| 80 | Yes | 2.0 | 44.5 | 850 |
| 80 | No | 3.7 | 27.3 | 3150 |

These results show that using an anti-pulsing device to achieve a uniform stream velocity produces a smaller median particle size, a higher respirable fraction from a standard dry powder inhaler and reduced residual solvent levels. It was also observed that the particle size distribution was narrower when an anti-pulsing device was used. This effect was especially marked at higher relative stream velocities.

## Claims

1. A method of producing medicament particles comprising dissolving the medicament in a solvent, producing one or more streams of medicament solution and contacting these streams with one or more streams of anti-solvent in order to produce a region of turbulent mixing in which rapid precipitation of medicament crystals takes place wherein the velocity of each stream is controlled to substantially remove cyclic variations and the ratio of the volume flow of anti-solvent to volume flow of medicament solution exceeds 2:1.

2. A method according to claim 1 in which the relative velocity of the streams exceeds 50m/s.

3. A method according to claim 1 or 2 in which the angle between the streams of solution and anti-solvent is less than 20°.

4. A method according to claim 1 or 2 in which the streams of solution and anti-solvent are substantially directly opposed.

5. A method according to any previous claim in which the relative velocity of the streams is between 70 and 200m/s.

6. A method according to any previous claim in which the ratio of volume flow of anti-solvent to medicament solution is greater than 10:1.

7. A method according to any one of claims 1 to 5 in which the ratio of volume flow of anti-solvent to medicament solution is between 15:1 and 30:1.

8. A method according to any previous claim in which the solvent is dimethylformamide.

9. A method according to any previous claim in which the anti-solvent is water.

10. A method according to any previous claim in which the medicament is triamcinolone acetonide.

11. An apparatus for carrying out a method according to any one of claims 1 to 9 comprising a cylinder with two or more orifices (3, 4) set into the cylinder walls through which streams of medicament solution and anti-solvent are produced which impinge on each other, and pumps which produce the streams of medicament solution and anti-solvent and where the apparatus comprises means for reducing cyclic variations in stream velocities.

12. An apparatus according to claim 11 in which the two or more orifices (3, 4) are arranged such that the angle between the streams of solution and anti-solvent is less than 20°.

13. An apparatus according to claim 11 in which the two or more orifices (3, 4) are arranged such that the streams of solution and anti-solvent are substantially directly opposed.

14. An apparatus according to any one of claims 11 to 13 in which the cylinder has an internal diameter between 0.2 and 1.0mm.

15. An apparatus according to any one of claims 11 to 14 in which the orifice (3) used to produce the medicament solution stream has a diameter between 50 and 200 micrometer and the orifice (4) used to produce the anti-solvent stream has a diameter between 100 and 500 micrometer.

## Patentansprüche

1. Verfahren zum Herstellen von Arzneimittelteilchen, umfassend Auflösen des Arzneimittels in einem Lösungsmittel, Herstellen von einem oder mehreren Strömen von Arzneimittellösung und In-Kontakt-Bringen dieser Ströme mit einem oder mehreren Strömen von Antilösungsmittel, um einen Bereich von turbulentem Vermischen zu erzeugen, worin schnelle Ausfällung von Arzneimittelkristallen stattfindet, wobei die Geschwindigkeit von jedem Strom gesteuert wird, um zyklische Änderungen im Wesentlichen zu beseitigen und das Verhältnis von dem Volumenstrom von Antilösungsmittel zum Volumenstrom von Arzneimittellösung 2:1 übersteigt.

2. Verfahren nach Anspruch 1, wobei die relative Geschwindigkeit der Ströme 50 m/s übersteigt.

3. Verfahren nach Anspruch 1 oder 2, wobei der Winkel zwischen den Strömen von Lösung und Antilösungsmittel weniger als 20° ist.

4. Verfahren nach Anspruch 1 oder 2, wobei die Ströme von Lösung und Antilösungsmittel im Wesentlichen direkt entgegengesetzt sind.

5. Verfahren nach einem vorangehenden Anspruch, wobei die relative Geschwindigkeit der Ströme zwischen 70 und 200 m/s ist.

6. Verfahren nach einem vorangehenden Anspruch, wobei das Verhältnis von Volumenstrom von Antilösungsmittel zu Arzneimittellösung größer als 10:1 ist.

7. Verfahren nach einem der Ansprüche 1 bis 5, wobei das Verhältnis von Volumenstrom von Antilösungsmittel zu Arzneimittellösung zwischen 15:1 und 30:1 ist.

8. Verfahren nach einem vorangehenden Anspruch, wobei das Lösungsmittel Dimethylformamid ist.

9. Verfahren nach einem vorangehenden Anspruch, wobei das Antilösungsmittel Wasser ist.

10. Verfahren nach einem vorangehenden Anspruch, wobei das Arzneimittel Triamcinolonacetonid ist.

11. Vorrichtung zum Ausführen eines Verfahrens nach einem der Ansprüche 1 bis 9, umfassend einen Zylinder mit zwei oder mehreren Öffnungen (3, 4), die in die Zylinderwände eingebracht sind, durch die Ströme von Arzneimittellösung und Antilösungsmittel erzeugt werden, welche aufeinander treffen, und Pumpen, die die Ströme von Arzneimittellösung und Antilösungsmittel erzeugen, und wobei die Vorrichtung Mittel zum Vermindern von zyklischen Änderungen von Stromgeschwindigkeiten umfasst.

12. Vorrichtung nach Anspruch 11, wobei die zwei oder mehreren Öffnungen (3, 4) derart angeordnet sind, dass der Winkel zwischen den Strömen von Lösung und Antilösungsmittel weniger als 20° ist.

13. Vorrichtung nach Anspruch 11, wobei die zwei oder mehreren Öffnungen (3, 4) derart angeordnet sind, dass die Ströme von Lösung und Antilösungsmittel im Wesentlichen direkt entgegengesetzt sind.

14. Vorrichtung nach einem der Ansprüche 11 bis 13, wobei der Zylinder einen Innendurchmesser zwischen 0,2 und 1,0 mm aufweist.

15. Vorrichtung nach einem der Ansprüche 11 bis 14, wobei die Öffnung (3), die zum Erzeugen des Arzneimittellösungsstroms verwendet wird, einen Durchmesser zwischen 50 und 200 Mikrometern aufweist, und die Öffnung (4), die zum Erzeugen des Antilösungsmittelstroms verwendet wird, einen Durchmesser zwischen 100 und 500 Mikrometern aufweist.

## Revendications

1. Procédé de préparation de particules de médicament consistant à dissoudre le médicament dans un solvant, produire un ou plusieurs courants de solution de médicament et mettre en contact ces courants avec un ou plusieurs courants d'anti-solvant afin de produire une région de mélange turbulent dans laquelle une précipitation rapide des cristaux de médicament se produit, dans laquelle la vitesse de chaque courant est contrôlée pour éliminer sensiblement les variations cycliques, et le rapport entre le débit de l'anti-solvant et le débit de la solution de médicament est supérieur à 2:1.

2. Procédé selon la revendication 1 dans lequel la vitesse relative des courants est supérieure à 50 m/s.

3. Procédé selon la revendication 1 ou la revendication 2 dans lequel l'angle entre les courants de la solution et de l'anti-solvant est inférieur à 20°.

4. Procédé selon la revendication 1 ou la revendication 2 dans lequel les courants de la solution et de l'anti-solvant sont pratiquement directement opposés.

5. Procédé selon l'une quelconque des revendications précédentes dans lequel la vitesse relative des courants est comprise entre 70 et 200 m/s.

6. Procédé selon l'une quelconque des revendications précédentes dans lequel le rapport entre le débit de l'anti-solvant et le débit de la solution de médicament est supérieur à 10:1.

7. Procédé selon l'une quelconque des revendications 1 à 5 dans lequel le rapport entre le débit de l'anti-solvant et le débit de la solution de médicament est compris entre 15:1 et 30:1.

8. Procédé selon l'une quelconque des revendications précédentes dans lequel le solvant est le diméthylformamide.

9. Procédé selon l'une quelconque des revendications précédentes dans lequel l'anti-solvant est l'eau.

10. Procédé selon l'une quelconque des revendications précédentes dans lequel le médicament est l'acétonide de triamcinolone.

11. Appareil destiné à effectuer un procédé selon l'une quelconque des revendications 1 à 9 comprenant un cylindre avec deux orifices ou plus (3, 4) fixés dans les parois du cylindre par lesquels sont produits les courants de la solution de médicament et de l'anti-solvant qui sont en contact l'un avec l'autre, et des pompes qui produisent les courants de la solution de médicament et de l'anti-solvant et lequel appareil comprend un moyen permettant de réduire les variations cycliques des vitesses de courant.

12. Appareil selon la revendication 11 dans lequel les deux orifices ou plus (3, 4) sont arrangés de sorte que l'angle entre les courants de la solution et de l'anti-solvant soit inférieur à 20°.

13. Appareil selon la revendication 11 dans lequel les deux orifices ou plus (3, 4) sont arrangés de sorte que les courants de la solution et de l'anti-solvant soient essentiellement directement opposés.

14. Appareil selon l'une quelconque des revendications 11 à 13 dans lequel le cylindre a un diamètre interne compris entre 0,2 et 1,0 mm.

15. Appareil selon l'une quelconque des revendications 11 à 14 dans lequel l'orifice (3) utilisé pour produire le courant de la solution de médicament a un diamètre compris entre 50 et 200 micromètres et l'orifice (4) utilisé pour produire le courant de l'anti-solvant a un diamètre compris entre 100 et 500 micromètres.
